Europäisches Patentamt

European Patent Office

Office européen des brevets

(11) Numéro de publication: **0 309 363**
**A1**

## (12) DEMANDE DE BREVET EUROPEEN

(21) Numéro de dépôt: **88402717.8**

(22) Date de dépôt: **03.04.87**

(51) Int. Cl.⁴: **A 61 F 2/36**

(30) Priorité: **04.04.86 FR 8604874**

(43) Date de publication de la demande:
**29.03.89 Bulletin 89/13**

(84) Etats contractants désignés:
**AT BE CH DE ES GB GR IT LI LU NL SE**

(60) Numéro de publication de la demande initiale en
application de l'article 76 CBE: **0 241 361**

(71) Demandeur: **Demeulenaere, Claude**
**Montplaisir 7 - 28, rue Wauthier**
**F-78100 St-Germain-en-Laye (FR)**

(72) Inventeur: **Demeulenaere, Claude**
**Montplaisir 7 - 28, rue Wauthier**
**F-78100 St-Germain-en-Laye (FR)**

(74) Mandataire: **Bruder, Michel**
**Cabinet Michel Bruder 10, rue de la Pépinière**
**F-75008 Paris (FR)**

(54) **Prothèse de hanche.**

(57) La présente invention concerne une prothèse de hanche.
Cette prothèse de hanche est caractérisée en ce qu'elle comporte un col fémoral (8) amovible et interchangeable.

*Fig. 9*

EP 0 309 363 A1

Bundesdruckerei Berlin

**Description**

La présente invention concerne une prothèse de hanche.

On utilise actuellement en chirurgie des prothèses de hanche qui sont constituées de trois pièces à savoir une cupule cotyloïdienne. une queue fémorale métallique comprenant un col et une tête fémorale sphérique solidaire ou indépendante du col.Ces prothèses peuvent être fixées à l'os soit par scellement grâce à du ciment plastique soit directement sans ciment par divers moyens plus ou moins valables.

Elles nécessitent toutes un creusement plus ou moins important de l'os qui fragilise ce dernier. en particulier au niveau du cotyle. De plus, le scellement au ciment peut entraîner des descellements au bout d'un temps variable.

La présente invention vise à améliorer les résultats des prothèses de hanche en proposant une prothèse cotyloïdienne non cimentée dont l'ancrage ne nécessite aucun creusement de l'os.

A cet effet cette prothèse de hanche comprenant une cupule cotyloïdienne externe métallique. de forme sensiblement hémisphérique. fixée dans l'os iliaque. un noyau cotyloïdien interne en matière plastique,emboité dans la cupule cotyloïdienne externe métallique, une queue fémorale emboîtée dans la diaphyse fémorale, une tête fémorale sphérique engagée dans le noyau cotyloïdien interne et un col de liaison entre la tête et la queue fémorale, est caractérisée en ce qu'elle comporte un col fémoral amovible et interchangeable.

Le col fémoral peut être coudé d'un angle variant de 10o à 30o. Cette disposition permet d'ajuster et éventuellement de corriger une antéversion ou d'une rétroversion de la queue fémorale.

On décrira ci-après à titre d'exemples non limitatifs, diverses formes d'exécution de la présente invention, en référence au dessin annexé sur lequel :

La figure 1 est une vue de profil d'une prothèse de hanche en place , dans le plan de l'aileron de la cupule cotyloïdienne externe.

La figure 2 est une vue de face de la prothèse de hanche en place.

La figure 3 est une vue de l'os iliaque, montrant le plan de coupe de la fente destinée à recevoir l'aileron.

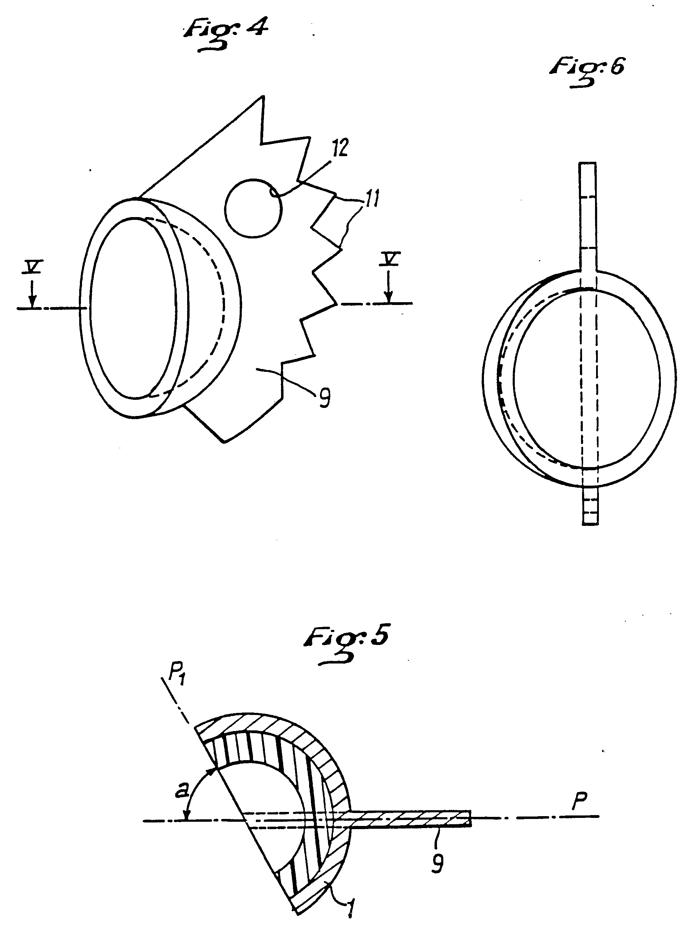

La figure 4 est une vue en élévation de la cupule cotyloïdienne externe métallique de la prothèse de hanche suivant l'invention, perpendiculairement au plan de l'aileron externe.

La figure 5 est une vue en coupe faite suivant la ligne V-V de la figure 4. le noyau cotyloïdien interne en polyéthylène étant représenté en place dans la cupule métallique externe.

La figure 6 est une vue de la cupule cotyloïdienne externe , prise de la gauche sur la figure 4 et regardant l'aileron par la tranche.

Les figures 7 et 8 sont des vues en coupe de divers noyaux cotyloïdiens en polyéthylène.

Les figures 9 et 10 sont des vues schématiques illustrant des cols fémoraux amovibles.

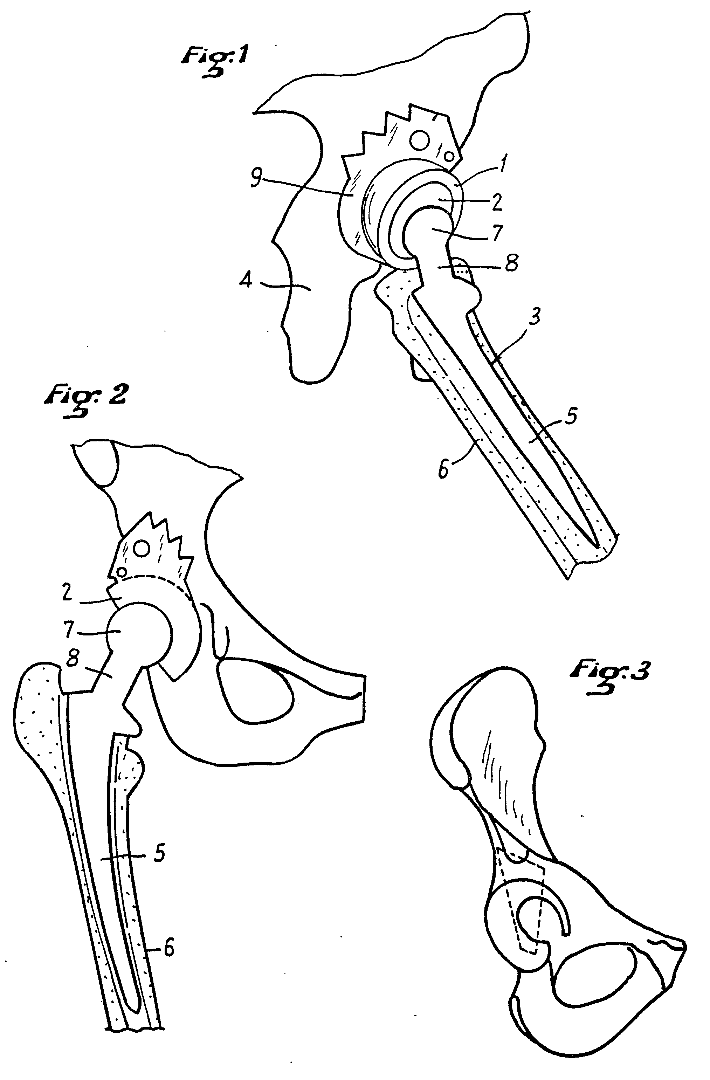

La prothèse de hanche suivant l'invention qui est représentée sur les figures 1 et 2, comprend essentiellement cinq pièces. à savoir une cupule cotyloïdienne externe métalliquel fixée dans l'os iliaque 4, un noyau cotyloïdien interne en matière plastique 2, emboité dans la cupule métallique externe 1, une queue fémorale 5 emboîtée dans la diaphyse fémorale une tête fémorale sphérique 7 logée dans le noyau cotyloïdien interne en matière plastique 2, et un col fémoral 8 assurant la liaison entre la tête 7 et la queue fémorale 5.

Suivant l'invention la cupule cotyloïdienne métallique externe 1 est encastrée dans 1 aile iliaque et dans l'ischion au moyen d'un aileron 9 qui s'étend vers la grande échancrure sciatique et qui est contenu dans un plan diamétral P (figure 5) de la cupule cotyloïdienne externe 1 hémisphérique. Ce plan P forme, avec le plan frontal P1 contenant l'ouverture du cotyle, un angle a qui est compris entre 50 et 80° et qui est de préférence égal à 65o. Cet aileron 9, de faible épaisseur, est fixé par emboîtement dans une fente de l'os de largeur sensiblement égale à l'épaisseur de l'aileron 9, fente dans laquelle il tient par simple frottement.

Pour améliorer la fixation de cet aileron d'ancrage 9 ,on peut prévoir, sur son bord périphérique 9, une découpe en dents de scie 11. Par ailleurs l'aileron 9 peut être percé d'un ou plusieurs trous 12 à travers lesquels peut se reconstituer l'os, en renforcant ainsi l'ancrage de la cupule cotyloïdienne 1.

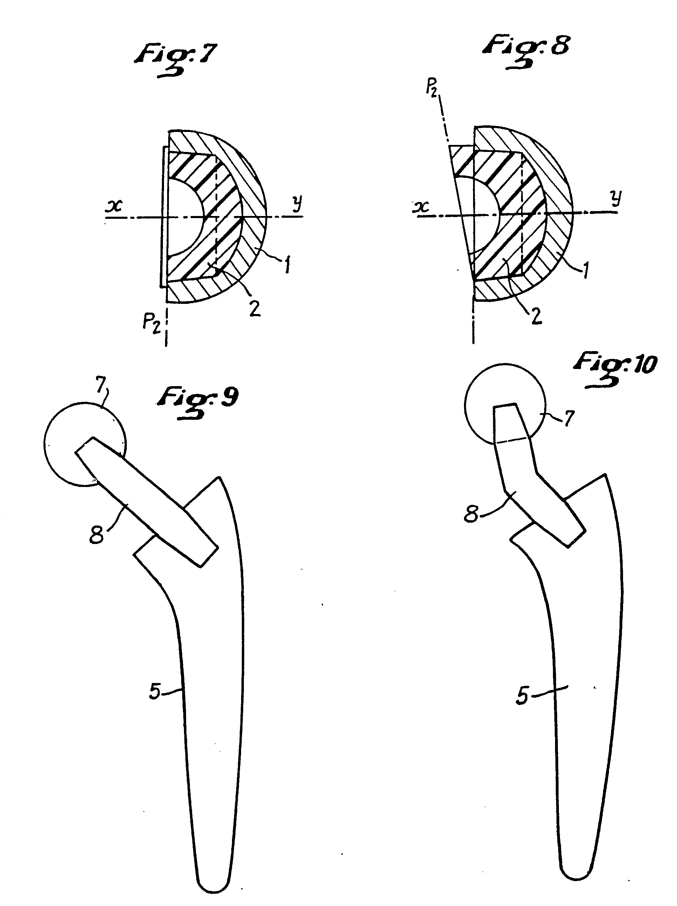

Suivant une caractéristique complémentaire de l'invention le noyau cotyloïdien interne 2 en polyéthylène présente un plan frontal d'antéversion, c'est-à-dire celui contenant l'ouverture du cotyle, qui fait un angle pouvant varier de 90o à 120o avec l'axe xy du noyau cotyloïdien comme il est représenté sur les figures 7 et 8. Cette disposition permet de mieux adapter la prothèse de hanche suivant l'invention aux conditions d'implantation particulières rencontrées.

La prothèse de hanche suivant l'invention comporte, ainsi qu'il est représenté sur les figures 9 et 10, un col fémoral 8 amovible indépendant de la queue fémorale 5 et donc interchangeable. Ce col fémoral interchangeable 8 peut être rectiligne, de longueur variable ainsi qu'il est représenté sur la figure 9, ou encore il peut être coudé, avec un angle allant de 10 à 30o, afin de permettre une modification de l'angle d'antéversion du col si nécessaire.

**Revendications**

1.- Prothèse de hanche comprenant une cupule cotyloïdienne externe métallique, de forme sensiblement hémisphérique fixée dans l'os iliaque , un noyau cotyloïdien interne en matière plastique, emboité dans la cupule cotyloïdienne externe métallique, une queue fémorale emboîtée dans la diaphyse fémorale , une tête fémorale sphérique engagée dans le noyau cotyloïdien interne et un col de liaison entre la tête et la queue fémorale, caractérisée en ce qu'elle comporte un col fémoral (8) amovible et interchangeable.

2.- Prothèse de hanche suivant la revendication 1 caractérisée en ce que le col fémoral interchangeable (8) est rectiligne.

3.- Prothèse de hanche suivant la revendication 1 caractérisée en ce que le col fémoral interchangeable (8) est coudé suivant un angle de 10 à 30o.

*Fig:1*

9

1

2

7

8

4

3

5

6

*Fig: 2*

2

7

8

5

6

*Fig:3*

EP 0 309 363 A1

Fig. 4

Fig. 6

Fig. 5

**Fig.7**

**Fig.8**

**Fig.9**

**Fig.10**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,P | EP-A-0 201 407 (MONTAGNE) <br> * Figure 1; page 5, line 12 - page 6, line 3 * | 1-3 | A 61 F 2/36 |
| X | US-A-3 840 904 (TRONZO) <br> * Figure 2; column 2, lines 37-42 * | 2-4 | |
| A | EP-A-0 099 167 (CARBOMEDICS INC.) <br> * Figures 1,2; page 8, lines 28-31 * | 2 | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 19-12-1988 | SEDY, R. |

EPO FORM 1503 03.82 (P0401)